# FASCICULE DE BREVET EUROPEEN

(11) **EP 4 507 764 B1**
(45) Date de publication et mention de la délivrance du brevet: **11.03.2026**
(21) Numéro de dépôt: 23722434.0
(22) Date de dépôt: 13.04.2023
(51) Int. Cl.: A61M 15/00, A61M 15/08

(54) **INHALATEUR DE POUDRE**
PULVERINHALATOR
POWDER INHALER

(30) Priorité: 14.04.2022 FR 2203475
(43) Date de publication de la demande: 19.02.2025
(73) Titulaire: APTAR France SAS, 27110 Le Neubourg (FR)
(72) Inventeur: BAILLET, Matthieu, 76000 Rouen (FR)
(74) Mandataire: CAPRI
(86) Numéro de dépôt international: PCT/FR2023/050531
(87) Numéro de publication internationale: WO 2023/198996

(56) Documents cités:
- WO-A1-2007/129128
- WO-A1-2007/144614
- WO-A1-2016/091386
- WO-A1-2019/101135
- WO-A2-2009/009013
- WO-A2-2012/004485

## Description

La présente invention concerne un inhalateur de poudre.

Les inhalateurs de poudre, notamment du type pharmaceutique, sont bien connus dans l'état de la technique. Il en existe différentes sortes. Un premier type d'inhalateur contient un réservoir recevant une multitude de doses de poudre, l'inhalateur étant pourvu de moyens de dosage permettant à chaque actionnement de séparer une dose de cette poudre du réservoir pour l'amener dans un conduit d'expulsion afin d'être distribué à l'utilisateur. Un autre type d'inhalateur consiste à disposer les doses de poudre dans des réservoirs individuels pré-dosés, puis d'ouvrir un de ces réservoirs à chaque actionnement de l'inhalateur. Cette mise en œuvre assure une meilleure étanchéité de la poudre, puisque chaque dose n'est ouverte qu'au moment de son expulsion. Pour réaliser ces réservoirs individuels, diverses variantes ont été proposées, telle qu'une bande de blisters allongée ou des blisters disposés sur un disque circulaire rotatif. Tous les types d'inhalateurs décrits ci-dessus et existants présentent des avantages et des inconvénients liés à leur structure et à leur fonctionnement. Les inhalateurs multidoses et les inhalateurs contenant plusieurs réservoirs individuels sont généralement des dispositifs complexes, constituées d'un grand nombre de pièces, et donc coûteux à fabriquer et à assembler.

Pour réaliser des dispositifs moins complexes et donc moins coûteux, il a été proposé des inhalateurs unidoses comportant un seul réservoir individuel, tel qu'une capsule ou un blister, qui est à charger dans l'inhalateur avant son utilisation. L'avantage de ces dispositifs est qu'il n'est pas nécessaire de stocker l'ensemble des doses à l'intérieur de l'appareil, de sorte que celui-ci peut être de dimension réduite.

Les documents WO2007129127, WO2007129128 et WO2012004485 décrivent des dispositifs de l'art antérieur. Ces dispositifs présentent des inconvénients, par exemple un assemblage peu aisé du réservoir individuel dans un inhalateur unidose, ce qui rend son utilisation compliquée par exemple en cas de crise ou pour des personnes handicapées. De même, ces dispositifs comportent généralement plusieurs pièces, ce qui complique la fabrication et l'assemblage.

Les documents WO2016091386 et WO2007144614 décrivent d'autres dispositifs de l'état de la technique. Le document WO2016091386 décrit un dispositif unidose en dorme de pince à linge, et le document WO2007144614 décrit un inhalateur oral.

Aucun de ces documents ne décrit un dispositif bidose adapté à distribuer deux doses successivement, par exemple une première dose dans une première narine et une seconde dose dans une seconde narine.

La présente invention a pour but de fournir un inhalateur de poudre, qui ne reproduit pas les inconvénients susmentionnés.

En particulier, la présente invention a pour but de fournir un tel inhalateur qui soit simple et peu coûteux à fabriquer et à assembler, et fiable d'utilisation.

La présente invention a donc pour objet un inhalateur de poudre, comportant un corps recevant un réservoir ayant une cavité contenant une dose de poudre et une couche de fermeture pour fermer ladite cavité de manière étanche, ledit corps comportant une partie de tête et deux parties de support, ladite partie de tête étant reliée à chaque partie de support par au moins une articulation, ladite partie de tête comportant un orifice de distribution et un élément de perforation relié audit orifice de distribution et adapté lors de l'actionnement à percer ladite couche de fermeture pour ouvrir ledit réservoir disposé sur une partie de support, une première partie de support recevant un premier réservoir contenant une première dose et une seconde partie de support recevant un second réservoir contenant une seconde dose, ledit corps étant réalisé d'une seule pièce monobloc.

Avantageusement, ledit orifice de distribution est formé à l'extrémité axiale d'un embout nasal destiné à être inséré dans une narine lors de l'actionnement.

Avantageusement, chaque articulation est formée par un pont de matière pliable.

Avantageusement, ladite partie de tête comporte au moins une fenêtre et chaque partie de support comporte au moins une projection pour coopérer en position fermée de l'inhalateur avec une fenêtre respective, notamment par encliquetage, pour verrouiller ladite position fermée.

Avantageusement, ledit verrouillage de la position fermée est déverrouillable, pour permettre la réutilisation du corps avec plusieurs réservoirs.

Avantageusement, chaque partie de support comporte un évidement adapté à loger ladite cavité dudit réservoir.

Avantageusement, ledit réservoir comporte au moins une ouverture de positionnement, chaque partie de support comportant au moins un plot recevant une ouverture de positionnement respective.

Avantageusement, après positionnement dudit réservoir sur une partie de support, chaque plot est déformé, notamment bouterollé, pour fixer ledit réservoir de manière non-amovible sur ladite partie de support.

Avantageusement, ladite partie de tête comporte au moins une patte de blocage pour bloquer l'inhalateur dans une position intermédiaire dans laquelle ledit élément de perforation n'a pas percé ladite couche de fermeture dudit réservoir, de sorte que lors de l'actionnement, l'utilisateur doit casser ou déformer ladite au moins une patte de blocage pour pouvoir actionner l'inhalateur.

Avantageusement, lesdites deux parties de support sont disposées de manière symétrique de part et d'autre de ladite partie de tête.

Ces caractéristiques et avantages et d'autres de la présente invention apparaîtront plus clairement au cours de la description détaillée suivante, faite en référence aux dessins joints, donnés à titre d'exemples non limitatifs, sur lesquels
la figure 1 est une vue schématique en perspective d'un inhalateur unidose de poudre selon un premier mode de réalisation avantageux, avant assemblage du réservoir et en position ouverte de l'inhalateur,
la figure 2 est une vue schématique en perspective d'un réservoir adaptable à l'inhalateur de la figure 1,
la figure 3 est une vue schématique en perspective similaire à celle de la figure 1, avec le réservoir assemblé dans l'inhalateur, en position ouverte de l'inhalateur,
la figure 4 est une vue similaire à celle de la figure 3, en cours de fermeture de l'inhalateur,
la figure 5 est une vue similaire à celle de la figure 4, en position fermée de l'inhalateur,
la figure 6 est une vue similaire à celle de la figure 3, montrant un inhalateur unidose de poudre selon un autre mode de réalisation avantageux,
la figure 7 est une vue similaire à celle de la figure 6, en position intermédiaire de l'inhalateur,
la figure 8 est une vue très schématique d'un inhalateur bidose de poudre selon un mode de réalisation avantageux de l'invention, en position ouverte de l'inhalateur avant sa première utilisation,
la figure 9 est une vue similaire à celle de la figure 8, montrant le pivotement de la première partie du support pour distribuer une première dose,
la figure 10 une vue similaire à celle de la figure 9, montrant le dispositif prêt pour la distribution de la première dose,
la figure 11 une vue similaire à celle de la figure 10, montrant le pivotement de la première partie du support après distribution de la première dose,
la figure 12 est une vue similaire à celle de la figure 11, montrant le pivotement de la seconde partie du support pour distribuer une seconde dose, et
la figure 13 une vue similaire à celle de la figure 12, montrant le dispositif prêt pour la distribution de la seconde dose.

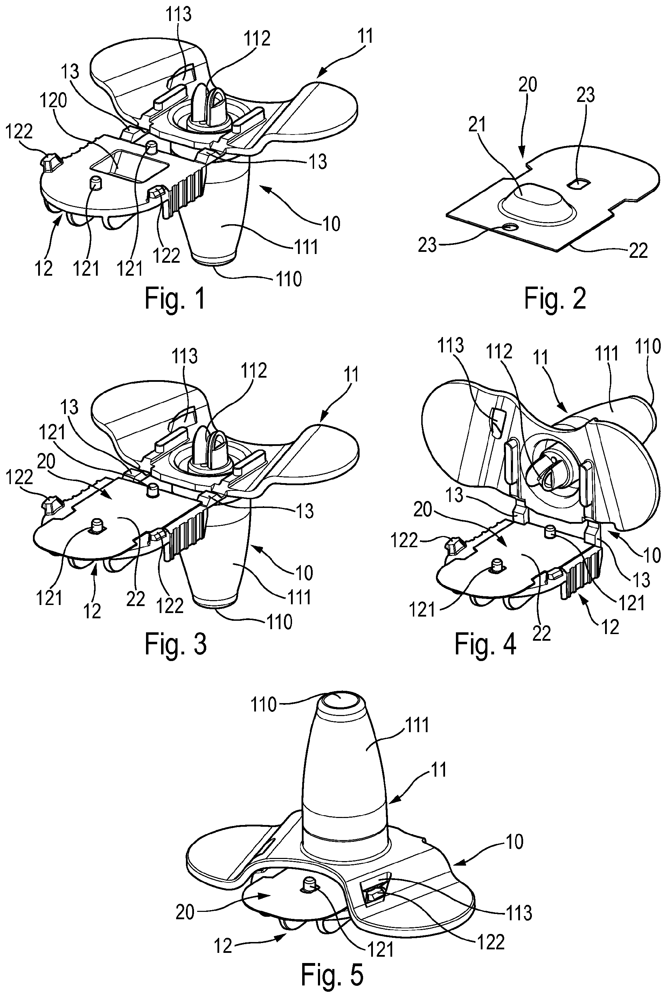

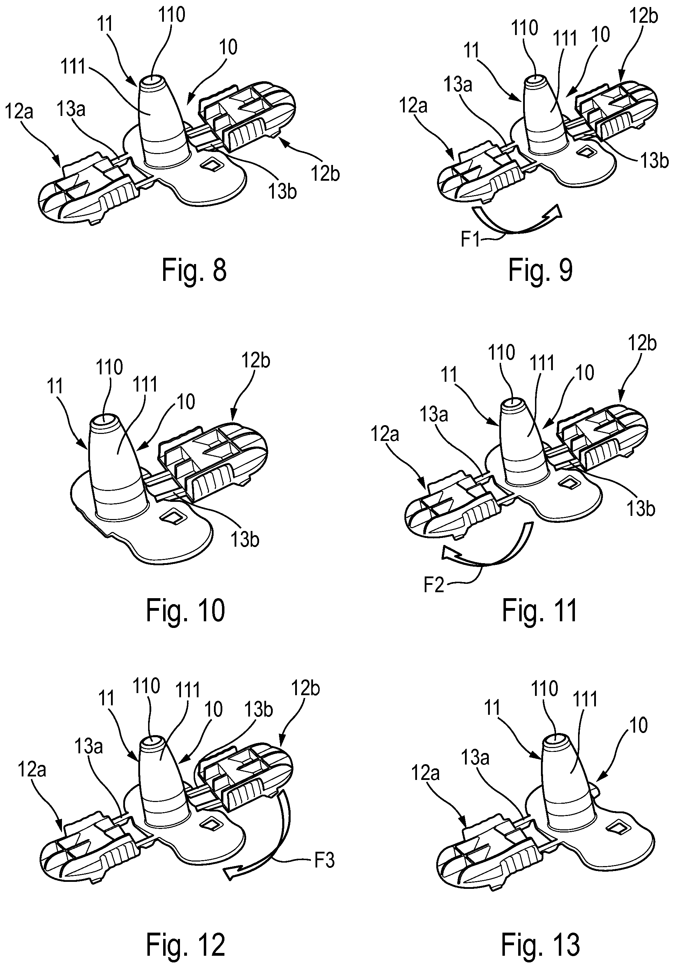

Les figures 1 à 7 décrivent un premier mode de réalisation avantageux, concernant un dispositif unidose. Ce premier mode de réalisation décrit de manière détaillée toutes les caractéristiques qui s'appliquent au mode de réalisation selon l'invention des figures 8 à 13.

L'inhalateur comporte un corps 10 monobloc réalisé d'une seule pièce par moulage d'une matière synthétique. Le corps 10 comporte une partie de tête 11 et une partie de support 12, lesdites parties 11, 12 étant reliées par au moins une articulation 13. Ainsi, le corps 10 est déformable entre une position ouverte, représentée sur les figures 1 et 3, et une position fermée, représentée sur la figure 5. De préférence, les parties de tête 11 et de support 12 pivotent par rapport à l'articulation 13 d'environ 180° entre les positions ouverte et fermée. Avantageusement, la ou les articulation(s) 13 est(sont) formée(s) par un ou plusieurs pont(s) de matière pliable(s).

La partie de tête 11 comporte un orifice de distribution 110, formé de préférence à l'extrémité axiale d'un embout nasal 111 que l'utilisateur place dans sa narine lors de l'utilisation de l'inhalateur. L'orifice de distribution 110 est relié à un élément de perforation 112. Avantageusement, la partie de tête comporte en outre au moins une fenêtre 113, avantageusement deux.

La partie de support 12 est destinée à recevoir un réservoir individuel 20, par exemple réalisé sous la forme d'un blister. Un exemple d'un tel réservoir 20 est représenté sur la figure 2. Ce réservoir 20 comporte une cavité 21 contenant une dose de poudre, et une couche de fermeture 22 pour fermer ladite cavité 21 de manière étanche. La couche de fermeture 22 est avantageusement réalisée en un matériau adapté à protéger la poudre contenue dans la cavité 21, notamment contre l'humidité, par exemple un métal tel que l'aluminium ou une structure multicouche appropriée. Le réservoir 20 comporte au moins une ouverture de positionnement 23, de préférence deux

La partie de support 12 comporte un évidement 120 adapté à loger la cavité 21 du réservoir 20. Elle comporte également au moins un plot 121, de préférence deux, pour recevoir les ouvertures de positionnement 23 du réservoir 20. Elle comporte aussi au moins une projection 122, de préférence deux, pour coopérer en position fermée de l'inhalateur avec les fenêtres 113 de la partie de tête 11, notamment par encliquetage, pour verrouiller ladite position fermée. Avantageusement, chaque projection 122 comporte un profil d'encliquetage qui va s'encliqueter dans une fenêtre respective 113 en position fermée, comme visible sur la figure 5.

Avantageusement, ce verrouillage peut être déverrouillé, ce qui permet de rouvrir l'inhalateur pour remplacer le réservoir vide par un nouveau réservoir plein, et ainsi réutiliser l'inhalateur pour distribuer plusieurs doses. Dans ce cas, les projections 122 peuvent avoir une forme différente, avec le profil d'encliquetage tourné vers l'intérieur, plutôt que vers l'extérieur comme dans les exemples des figures 1 à 7.

Lorsque l'utilisateur souhaite utiliser l'inhalateur, il prend le corps 10 en position ouverte et positionne un réservoir 20 sur la partie de support 12. La cavité 21 du réservoir 20 est disposée dans l'évidement 120 et les ouvertures de positionnement 23, au nombre de deux dans l'exemple représenté, sont disposées autour des plots 121, également au nombre de deux. La couche de fermeture 22 est donc tournée vers le haut, dans la position telle que représentée sur la figure 3.

L'utilisateur ferme alors le corps 10 en pivotant d'environ 180° la partie de tête 11 sur la partie de support 12 autour des articulations 13, au nombre de deux dans l'exemple représenté. Pendant ce mouvement de fermeture, l'élément de perforation 112 va percer la couche de fermeture 22 du réservoir 20 et pénétrer dans la cavité 21. En position fermée, les projections 122 de la partie de support 12 s'encliquète dans les fenêtres 113 de la partie de tête 11 pour verrouiller cette position fermée lors de l'actionnement.

L'utilisateur place alors l'embout nasal 111 dans une narine et aspire, ce qui distribue la poudre contenue dans la cavité 21 à travers l'élément de perforation 112 et l'orifice de distribution 110.

Si l'inhalateur est à usage unique, il est jeté. Dans ce cas, le verrouillage de la position fermée est de préférence définitif. Si au contraire l'inhalateur est réutilisable, l'utilisateur déverrouille la position fermée et ramène le corps 10 en position ouverte, dans laquelle le réservoir vide peut être retiré de la partie de support 12. Avantageusement, le corps 10, en particulier la partie de tête 11, peut être nettoyé ou lavé avant une autre utilisation.

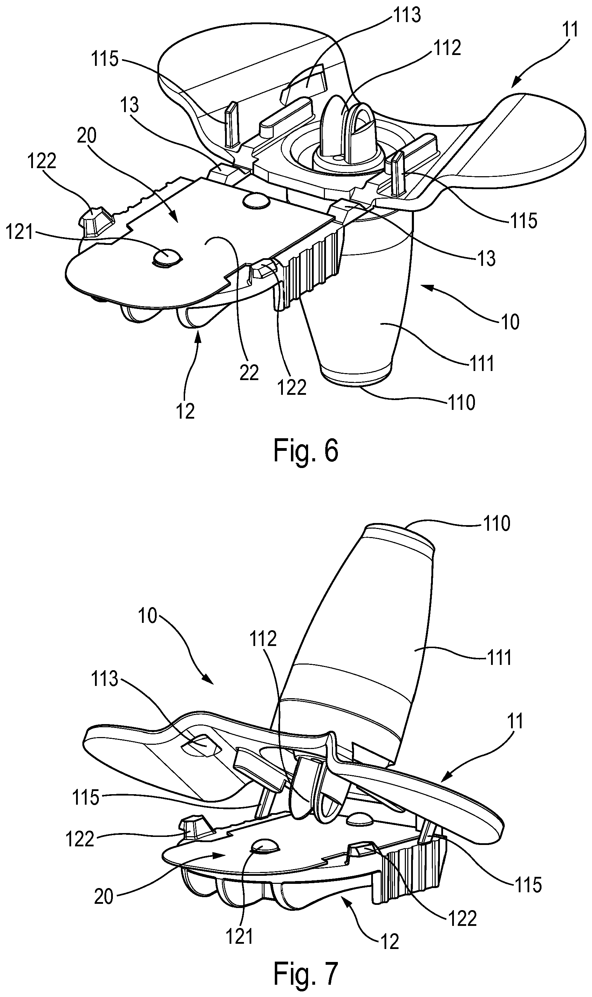

Dans la variante de réalisation des figures 6 et 7, le réservoir 20 peut être fixé sur la partie de support 12 de manière non-amovible. Par exemple, après avoir disposé le réservoir 20 sur la partie de support 12, avec la cavité 21 disposée dans l'évidement 120 et les ouvertures de positionnement 23 disposés autour des plots 121, ces derniers peuvent être déformés, par exemple par bouterollage. De cette manière, les plots 121 s'élargissent et viennent coincer le réservoir 20 sur la partie de support. En variante, on pourrait envisager un encliquetage des ouvertures de positionnement 23 sur les plots 121, qui pourraient alors avoir un profil adapté.

Cette mise en œuvre permet de préinstaller le réservoir 20 dans l'inhalateur, et de livrer l'ensemble ainsi préassemblé. L'utilisateur n'a alors plus besoin de positionner un réservoir sur la partie de support 12 avant d'utiliser l'inhalateur, mais seulement de refermer ce dernier.

La partie de tête 11 dans l'exemple des figures 6 et 7 peut aussi comporter au moins une patte de blocage 115, de préférence deux, pour bloquer l'inhalateur dans une position intermédiaire dans laquelle l'élément de perforation 112 n'a pas percé la couche de fermeture 22 du réservoir 20. Cette position intermédiaire est visible sur la figure 7. Dans cette position intermédiaire, l'extrémité de chaque patte de blocage 115 bute sur une surface de ladite partie de support 12. Lors de l'actionnement, l'utilisateur doit alors casser ou déformer ces pattes de blocage 115 pour pouvoir actionner l'inhalateur. Dans cet exemple, le dispositif peut être livré en position intermédiaire, avec le réservoir 20 préinstallé, et l'utilisateur pour actionner n'a alors besoin que d'appuyer suffisamment fort sur le corps 10 pour casser ou déformer les pattes de blocage 115 et ainsi amener le corps 10 en position fermée, avec l'élément de perforation 112 qui a percé la couche de fermeture 22 du réservoir pour permettre l'inhalation de la dose de poudre.

Les figures 8 à 13 décrivent de manière très schématique un mode de réalisation avantageux de l'invention, concernant un dispositif bidose. Ce mode de réalisation correspond à une version bidose du dispositif décrit dans le premier mode de réalisation, et toutes les caractéristiques et variantes décrites dans le cadre de ce premier mode de réalisation en référence aux figures 1 à 7 s'appliquent à ce mode de réalisation.

Selon l'invention, le corps 10 comporte deux parties de support 12a, 12b, chacune étant reliée à la partie de tête 11 par au moins une articulation 13a, 13b. De préférence, ces deux parties de support 12a, 12b sont disposées de manière symétrique de part et d'autre de la partie de tête, et elles sont avantageusement identiques.

La première partie de support 12a reçoit un premier réservoir contenant une première dose et la seconde partie de support 12b reçoit un second réservoir contenant une seconde dose.

Pour distribuer la première dose, l'utilisateur pivote d'environ 180° la première partie de support 12a comportant son réservoir respectif, comme illustré par la flèche F1 sur la figure 9, pour distribuer la première dose.

Après distribution de la première dose, il ramène la première partie de support 12a dans sa position initiale, comme illustré par la flèche F2 sur la figure 11, puis pivote d'environ 180° la seconde partie de support 12b comportant son réservoir respectif, comme illustré par la flèche F3 sur la figure 12, pour distribuer la seconde dose.

Diverses modifications sont possibles pour un homme du métier sans sortir du cadre de la présente invention tel que défini par les revendications annexées.

## Revendications

1. Inhalateur de poudre, comportant un corps (10) recevant un réservoir (20) ayant une cavité (21) contenant une dose de poudre et une couche de fermeture (22) pour fermer ladite cavité (21) de manière étanche, ledit corps (10) comportant une partie de tête (11) et deux parties de support (12a, 12b), ladite partie de tête (11) étant reliée à chaque partie de support (12a, 12b) par au moins une articulation (13a, 13b), ladite partie de tête (11) comportant un orifice de distribution (110) et un élément de perforation (112) relié audit orifice de distribution (110) et adapté lors de l'actionnement à percer ladite couche de fermeture (22) pour ouvrir ledit réservoir (20) disposé sur une partie de support (12a, 12b), une première partie de support (12a) recevant un premier réservoir contenant une première dose et une seconde partie de support (12b) recevant un second réservoir contenant une seconde dose, ledit corps (10) étant réalisé d'une seule pièce monobloc.

2. Inhalateur selon la revendication 1, dans lequel ledit orifice de distribution (110) est formé à l'extrémité axiale d'un embout nasal (111) destiné à être inséré dans une narine lors de l'actionnement.

3. Inhalateur selon la revendication 1 ou 2, dans lequel chaque articulation (13a, 13b) est formée par un pont de matière pliable.

4. Inhalateur selon l'une quelconque des revendications précédentes, dans lequel ladite partie de tête (11) comporte au moins une fenêtre (113) et chaque partie de support (12a, 12b) comporte au moins une projection (122) pour coopérer en position fermée de l'inhalateur avec une fenêtre (113) respective, notamment par encliquetage, pour verrouiller ladite position fermée.

5. Inhalateur selon la revendication 4, dans lequel ledit verrouillage de la position fermée est déverrouillable, pour permettre la réutilisation du corps avec plusieurs réservoirs (20).

6. Inhalateur selon l'une quelconque des revendications précédentes, dans lequel chaque partie de support (12a, 12b) comporte un évidement (120) adapté à loger ladite cavité (21) dudit réservoir (20).

7. Inhalateur selon l'une quelconque des revendications précédentes, dans lequel ledit réservoir (20) comporte au moins une ouverture de positionnement (23), chaque partie de support (12) comportant au moins un plot (121) recevant une ouverture de positionnement (23) respective.

8. Inhalateur selon la revendication 7, dans lequel après positionnement dudit réservoir (20) sur une partie de support (12a, 12b), chaque plot (121) est déformé, notamment bouterollé, pour fixer ledit réservoir (20) de manière non-amovible sur ladite partie de support (12a, 12b).

9. Inhalateur selon l'une quelconque des revendications précédentes, dans lequel ladite partie de tête (11) comporte au moins une patte de blocage (115) pour bloquer l'inhalateur dans une position intermédiaire dans laquelle ledit élément de perforation (112) n'a pas percé ladite couche de fermeture (22) dudit réservoir (20), de sorte que lors de l'actionnement, l'utilisateur doit casser ou déformer ladite au moins une patte de blocage (115) pour pouvoir actionner l'inhalateur.

10. Inhalateur selon l'une quelconque des revendications précédentes, dans lequel lesdites deux parties de support (12a, 12b) sont disposées de manière symétrique de part et d'autre de ladite partie de tête (11).

## Patentansprüche

1. Pulverinhalator, aufweisend einen Körper (10), der einen Behälter (20) mit einem Hohlraum (21) aufnimmt, der eine Pulverdosis enthält, und einer Verschlussschicht (22) zum dichten Verschließen des Hohlraums (21), wobei der Körper (10) einen Kopfteil (11) und zwei Stützteile (12a, 12b) aufweist, wobei der Kopfteil (11) mit jedem Stützteil (12a, 12b) über mindestens ein Gelenk (13a, 13b) verbunden ist, wobei der Kopfteil (11) eine Abgabeöffnung (110) und ein mit der Abgabeöffnung (110) verbundenes Durchstechelement (112) aufweist, das bei Betätigung dazu geeignet ist, die Verschlussschicht (22) zu durchstechen, um den auf einem Stützteil (12a, 12b) angeordneten Behälter (20) zu öffnen, wobei ein erster Stützteil (12a) einen ersten Behälter aufnimmt, der eine erste Dosis enthält, und ein zweiter Stützteil (12b) einen zweiten Behälter aufnimmt, der eine zweite Dosis enthält, wobei der Körper (10) aus einem einzigen einteiligen Stück gefertigt ist.

2. Inhalator nach Anspruch 1, wobei die Abgabeöffnung (110) am axialen Ende eines Nasenstücks (111) ausgebildet ist, das bei Betätigung zum Einführen in ein Nasenloch bestimmt ist.

3. Inhalator nach Anspruch 1 oder 2, wobei jedes Gelenk (13a, 13b) durch eine Brücke aus biegsamem Material gebildet ist.

4. Inhalator nach einem der vorstehenden Ansprüche, wobei der Kopfteil (11) mindestens ein Fenster (113) aufweist und jeder Stützteil (12a, 12b) mindestens einen Vorsprung (122) aufweist, um in der geschlossenen Position des Inhalators mit einem jeweiligen Fenster (113) zusammenzuwirken, insbesondere durch Einrasten, um die geschlossene Position zu verriegeln.

5. Inhalator nach Anspruch 4, wobei die Verriegelung der geschlossenen Position entriegelbar ist, um die Wiederverwendung des Körpers mit mehreren Behältern (20) zu ermöglichen.

6. Inhalator nach einem der vorstehenden Ansprüche, wobei jeder Stützteil (12a, 12b) eine Aussparung (120) aufweist, die zur Aufnahme des Hohlraums (21) des Behälters (20) geeignet ist.

7. Inhalator nach einem der vorstehenden Ansprüche, wobei der Behälter (20) mindestens eine Positionierungsöffnung (23) aufweist, wobei jeder Stützteil (12) mindestens eine Erhebung (121) aufweist, der eine jeweilige Positionierungsöffnung (23) aufnimmt.

8. Inhalator nach Anspruch 7, wobei nach dem Positionieren des Behälters (20) auf einem Stützteil (12a, 12b) jede Erhebung (121) verformt, insbesondere gestaucht wird, um den Behälter (20) unlösbar auf dem Stützteil (12a, 12b) zu befestigen.

9. Inhalator nach einem der vorstehenden Ansprüche, wobei der Kopfteil (11) mindestens eine Arretierlasche (115) aufweist, um den Inhalator in einer Zwischenposition zu arretieren, in der das Durchstechelement (112) die Verschlussschicht (22) des Behälters (20) nicht durchstochen hat, so dass der Benutzer bei der Betätigung die mindestens eine Arretierlasche (115) brechen oder verformen muss, um den Inhalator betätigen zu können.

10. Inhalator nach einem der vorstehenden Ansprüche, wobei die beiden Stützteile (12a, 12b) symmetrisch zu beiden Seiten des Kopfteils (11) angeordnet sind.

## Claims

1. Powder inhaler, comprising a body (10) for receiving a reservoir (20) having a cavity (21) containing a dose of powder and a closing layer (22) to sealingly close said cavity (21), said body (10) comprising a head portion (11) and two support portions (12a, 12b), said head portion (11) being connected to each support portion (12a, 12b) by at least one joint (13a, 13b), said head portion (11) having a dispensing opening (110) and a perforating element (112) connected to said dispensing opening (110), which perforating element is designed, when actuated, to pierce said closing layer (22) to open said reservoir (20) arranged on a support portion (12a, 12b), a first support portion (12a) for receiving a first reservoir containing a first dose and a second support portion (12b) for receiving a second reservoir containing a second dose, said body (10) being formed of a single piece.

2. Inhaler according to claim 1, wherein said dispensing opening (110) is formed at the axial end of a nasal endpiece (111) intended to be inserted into a nostril during actuation.

3. Inhaler according to claim 1 or 2, wherein each joint (13a, 13b) is formed by a bridge of foldable material.

4. Inhaler according to any one of the preceding claims, wherein said head portion (11) has at least one window (113) and each support portion (12a, 12b) has at least one projection (122) for engaging in the closed position of the inhaler with a respective window (113), in particular by snap-fitting, to lock said closed position.

5. Inhaler according to claim 4, wherein said locking of the closed position is unlockable, to enable the body to be reused with several reservoirs (20).

6. Inhaler according to any one of the preceding claims, wherein each support portion (12a, 12b) has a recess (120) adapted to house said cavity (21) of said reservoir (20).

7. Inhaler according to any one of the preceding claims, wherein said reservoir (20) has at least one positioning opening (23), each support portion (12) having at least one stud (121) for receiving a respective positioning opening (23).

8. Inhaler according to claim 7, wherein after positioning said reservoir (20) on a support portion (12a, 12b), each stud (121) is deformed, in particular snapped, to non-removably fix said reservoir (20) on said support portion (12a, 12b).

9. Inhaler according to any one of the preceding claims, wherein said head portion (11) has at least one blocking tab (115) for locking the inhaler in an intermediate position in which said perforating element (112) has not pierced said closing layer (22) of said reservoir (20), such that during actuation, the user must break or deform said at least one blocking tab (115) in order to be able to actuate the inhaler.

10. Inhaler according to any one of the preceding claims, wherein said two support portions (12a, 12b) are arranged symmetrically on either side of said head portion (11).
